# EUROPEAN PATENT SPECIFICATION

(11) **EP 0 540 217 B1**
(45) Date of publication and mention of the grant of the patent: **13.05.1998**
(21) Application number: 92309509.5
(22) Date of filing: 19.10.1992
(51) Int. Cl.: A61M 5/32, A61M 25/06, A61B 5/14

(54) **Holder for double-ended needles**
Halter für doppelendige Nadeln
Support pour aiguilles à deux bouts

(30) Priority: 29.10.1991 US 783825
(43) Date of publication of application: 05.05.1993
(73) Proprietor: SAFETY SYRINGES, INC., South Pasadena, California 91030 (US)
(72) Inventor: Firth, John R., Wilsonville, Oregon 97070 (US); Perez, Anthony R., Alhambra, California 91801 (US); Meyer, Ronald A.., San Dimas, California 91773 (US)
(74) Representative: Parker, Nigel Edward

(56) References cited:
- EP-A- 0 281 421
- EP-A- 0 382 190
- US-A- 3 890 971
- US-A- 4 892 521

## Description

This invention relates to a medical fixture and more particularly to a holder for needles.

The improvements in this patent specification include features to prevent medical practitioners and other users from being stuck by needles after they have been in contact with body fluids from patients, and more particularly includes:
a movable shield which is positioned over a VACUTAINER^{R} holder needle.

### BACKGROUND OF THE INVENTION

The development of safe medical syringes and procedures for using them have long been a matter of concern, especially to the medical field. Many medical procedures, such as the transfusion of blood and the administration of insulin in some circumstances, require the use of needles and syringes, with their attendant hazard of accidental scratch or puncture. Recently, the risk has increased dramatically of contracting virulent and fatal or near-fatal infections during legitimate medical procedures using needles, and the general public as well as the medical profession has demanded safer products and procedures.

Efforts to minimize the risk of accidental infection have been made for several decades. Some representative efforts are:

La Marche U.S. Patent 1,921,034 discloses a protector case for a syringe of conventional type, which allows for the needle to be exposed the correct amount for the type of shot to be given, and to be retracted after use to protect others from being accidentally punctured thereby.

Bastien US-A-2,571,653 discloses a syringe body encased in a slidable protector case, having built-in detents to hold the needle in a retracted position prior to and following use, while permitting the needle to be exposed the correct length for the purpose.

Tschischeck US-A-2,586,581 discloses an add-on magnifying attachment for syringes. It has no relevance to the present invention, except to disclose one means of making the scale more readable and the dosage more accurate.

Norgren US-A-3,943,927 discloses an injection device to be carried by persons having allergic reactions to insect or snake bites, or bee stings, where antitoxins or other emergency remedies need to be administered immediately under conditions of great physical or emotional stress. It has little relevance to the present invention.

Ethington US-A-4,018,223 discloses a tactile-detent dosage metering device for use by persons with impaired vision.

Chen US-A-4,728,321 discloses a means to render a needle unfit for further use, by placing thereon a syringe cap and permanently cementing it in place.

Bogan US-A-4,738,663 discloses a removable protective cover for the needle of a syringe.

Leopoldi et al US-A-4,743,234 discloses a syringe with needle shield which provides protection to users when the shield is moved to cover the needle. The mechanism provides detent positions in both extended and retracted positions, but not a permanently locked position.

U.S. Patent Specification No. US-A-4892521 appears to show a protective hypodermic needle cover assembly having the features of the pre-characterising portion of Claim 1 of this specification.

None of the above references disclose, either singly or in combination, the structure and attendant features of the present invention, or those of the improvements in this patent specification.

### BRIEF DESCRIPTION OF THE PRESENT INVENTION

According to the present invention there is provided a holder for needles, said holder having a movable protective shield, comprising:
a. a needle guard having a U-shape, including an annular face plate extending radially and having an axial aperture therein with a collar therearound to permit a first end of a needle to extend therethrough, and longitudinal extensions on said collar extending rearwardly therefrom, and having tabs on the ends thereof extending laterally outwardly therefrom;
b. an extended body having a longitudinal cylindrical wall and a closed end with a hub thereon to removably receive said needle, and openings radially spaced from said hub, and an open end remote from said closed end;
   characterised by the holder being a safety holder adapted for receiving a double-ended needle and the longitudinal cylindrical wall being adapted for receiving and holding a vacuum-sealed fluid holder insertable therein to allow a second end of the needle to penetrate the fluid holder for transfer of fluid through the needle to or from the fluid holder, the cylindrical wall being provided with spaced longitudinal channels for slidingly receiving said longitudinal extensions and said openings adjoining or being aligned with the channels and permitting the longitudinal extensions and the tabs to be inserted within the cylindrical wall into the associated longitudinal channels, said channels having therein lateral apertures to receive said tabs in a releasable locking manner when said first end of a needle is surrounded by said collar.

The holder may have longitudinal extensions which comprise a pair of relatively flat, elongated extensions for fitting and sliding within the longitudinal channels and having a substantially slidably mating configuration with said channels to allow said extensions to slide back and forth within the channels without obstructing the interior of the body from receiving a standard vacuum-sealed fluid holder.

The lateral apertures may be disposed approximately midway between the closed and open ends of the body, and are substantially rectangular in shape to receive substantially mating tabs of similar substantially rectangular shape.

The protective case feature is incorporated in the double-needle fixture, for use when multiple samples of a patient's blood, are to be obtained at the same time. A nurse or other medical person is at a substantial risk using a fixture of conventional design in this situation. With an unprotected-needle fixture, one hand must be occupied in holding the first needle in a patient's arm while the other hand must withdraw one container from the second needle and reinsert another thereon, perhaps several times, leaving the needle exposed each time. Or, even a fixture with a protected needle requires that the collecting container be screwed onto the fixture, requiring precise manipulation in an awkward situation. With the present invention, the needle is covered by the protective case while one container is easily exchanged for another.

The movable shield for a standard VACUTAINER^{R.} holder may reduce the possibility of accidental needle sticks.

### BRIEF DESCRIPTION OF THE DRAWINGS

FIGURE 1 is an oblique view of a needle-shielding VACUTAINER^{R} holder in accordance with the present invention, with the needle thereof extended.

FIGURE 2 is an oblique view of the needle-shielding VACUTAINER^{R} holder as shown in FIGURE 1, with the needle thereof retracted.

FIGURE 3 is an oblique view of the VACUTAINER^{R} holder body member.

FIGURE 4 is an oblique view of the VACUTAINER^{R} holder slider member.

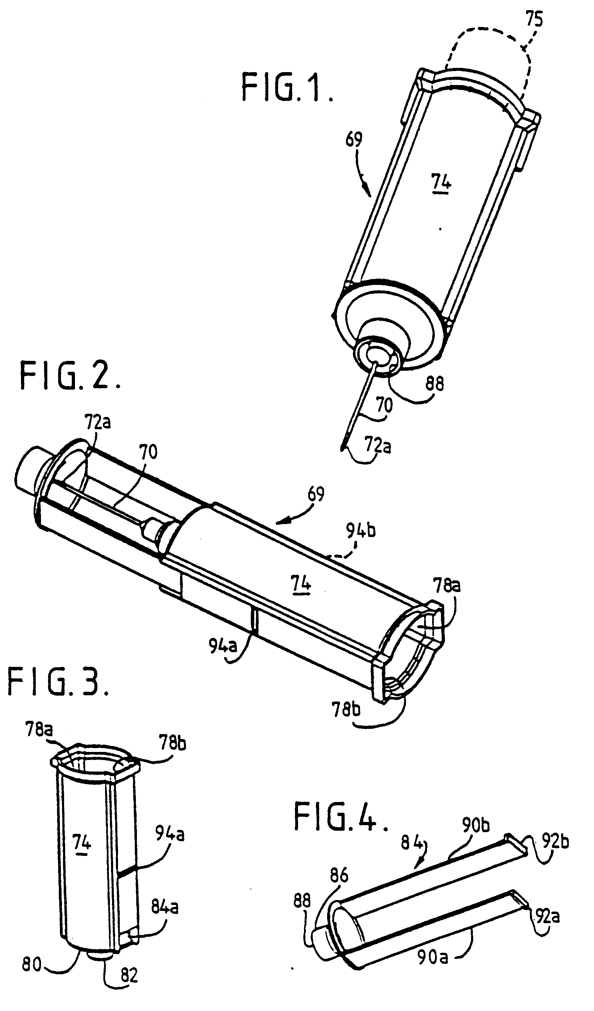

As FIGURES 1 to 4 disclose a fluid collection medical fixture in the form of safety holder 69 for double-ended syringe needles 70, greatly reduces the possibility of accidental injury by needle stick to medical personnel. Double-ended needle 70, having points 72a and 72b (not shown) thereon, is attached to longitudinal cylindrical body 74, the interior of which is sized to receive a vacuum-sealed fluid container 75 (i.e., the widely-used VACUTAINER^{R} container, shown in dashed outline). The rubber stopper thereof is inserted first so that it will be pierced by needle point 72b (not shown) of double-ended needle 70. Body 74 also has closed end 80, which includes hub 82 for mounting needle 70 thereon, hub 82 incorporating any of the structures by which needles can be attached thereto. Body 74 has formed in the sides thereof longitudinal channels 78a and 78b. Closed end 80 also has on the periphery thereof openings 84a and 84b (not shown) opening into channels 78a and 73b.

Slidingly associated with body 74 is needle guard 84, having a U-shape, and including annular face 86 thereon, which has collared aperture 88 therein for allowing needle 70 to extend therethrough. On the periphery of annular face 86 are longitudinal extensions 90a and 90b which, extending rearwardly from face 86 through openings 84a and 84b, slide in channels 78a and 78b, respectively. On the ends of extensions 90a and 90b are tabs 92a and 92b, respectively, which extend laterally therefrom and, when needle guard 84 is pulled forward to cover needle point 72a, snap into apertures 94a and 94b when needle point 72a is surrounded and protected by annular face 86 and collar 88, making it difficult for as user to be injured thereby.

In use, safety holder 69 comes with needle guard 84 extended, as shown in FIGURE 2. When it is desired to transfer fluid (blood for example) from sealed container 75 to another container, the rubber stopper end of container 75 is inserted into cylindrical body 74, and pressed onto needle point 72b. Longitudinal extension 90a and 90b are pressed inwardly, releasing tabs 92a and 92b from apertures 94a and 94b, permitting needle guard 84 to be slid rearwardly, exposing needle 70 and permitting it to be inserted into another container. When the fluid is transferred, longitudinal extensions 90a and 90b are pressed inwardly, permitting needle guard 84 to be slid forward, withdrawing needle 70 from the container and covering up needle point 72a at the same time, rendering it safe.

## Claims

1. A holder (69) for needles (70), said holder (69) having a movable protective shield (84), comprising:
a. a needle guard (84) having a U-shape, including an annular face plate (86) extending radially and having an axial aperture therein with a collar (88) therearound to permit a first end (72a) of a needle (70) to extend therethrough, and longitudinal extensions (90a,b) on said collar (88) extending rearwardly therefrom, and having tabs (92a,b) on the ends thereof extending laterally outwardly therefrom;
b. an extended body (74) having a longitudinal cylindrical wall and a closed end (80) with a hub (82) thereon to removably receive said needle (70), and openings (84a,b) radially spaced from said hub, and an open end remote from said closed end (80);
characterised by the holder (69) being a safety holder (69) adapted for receiving a double-ended needle (70) and the longitudinal cylindrical wall being adapted for receiving and holding a vacuum-sealed fluid holder insertable therein to allow a second end of the needle to penetrate the fluid holder for transfer of fluid through the needle to or from the fluid holder, the cylindrical wall being provided with spaced longitudinal channels (78a,78b) for slidingly receiving said longitudinal extensions (90a,b) and said openings (84a,b) adjoining or being aligned with the channels (78a,78b) and permitting the longitudinal extensions (90a,b) and the tabs (92a,b) to be inserted within the cylindrical wall into the associated longitudinal channels (78a,b), said channels (78a,b) having therein lateral apertures (94a,b) to receive said tabs (92a,b) in a releasable locking manner when said first end (72a) of a needle (70) is surrounded by said collar (88).

2. A holder (69) as claimed in Claim 1 wherein the longitudinal extensions (90a,b) comprise a pair of relatively flat, elongated extensions for fitting and sliding within the longitudinal channels (7a,b) and having a substantially slidably mating configuration with said channels (7a,b) to allow said extensions to slide back and forth within the channels without obstructing the interior of the body (74) from receiving a standard vacuum-sealed fluid holder.

3. A holder (69) as claimed in Claim 1 wherein the lateral apertures (94a,b) are disposed approximately midway between the closed and open ends of the body (74), and are substantially rectangular in shape to receive substantially mating tabs (92a,b) of similar substantially rectangular shape.

## Patentansprüche

1. Halter (69) für Nadeln (70), welcher Halter (69) einen beweglichen Schutzschild (84) aufweist, umfassend:
a. einen Nadelschutz (84) mit einer U-förmigen Gestalt, welcher eine ringförmige Stirnplatte (86), die sich in radialer Richtung erstreckt und darin eine axiale Öffnung mit einem diese umgebenden Kragen (88) hat und ermöglicht, daß sich ein erstes Ende (72a) einer Nadel (70) dort hindurcherstrecken kann, und längliche Fortsätze (90a, b) an dem Kragen (88) umfaßt, welche sich von dem Kragen weg nach hinten erstrecken und an ihren Enden sich seitlich nach außen erstreckende Vorsprünge (92a, b) aufweisen;
b. einen gestreckten Körper (74) mit einer länglichen zylindrischen Wand und einem geschlossenen, eine Nabe (82) aufweisenden Ende (80) zum abnehmbaren Aufnehmen der Nadel (70) sowie mit in radialer Richtung von der Nabe beabstandeten Öffnungen (84a, b) und mit einem dem geschlossenen Ende (80) gegenüberliegenden offenen Ende;
**dadurch gekennzeichnet,** dass der Halter (69) ein zum Aufnehmen einer doppelendigen Nadel (70) geeigneter Sicherheitshalter (69) ist und die längliche zylindrische Wand zum Aufnehmen und Halten eines darin einsetzbaren vakuumdichten Fluidhalters geeignet ist, damit ein zweites Ende der Nadel zum Übertragen des Fluids durch die Nadel in den oder aus dem Fluidhalter eindringen kann, daß die zylindrische Wand mit voneinander beabstandeten, länglichen Kanälen (78a, 78b) zum gleitenden Aufnehmen der länglichen Fortsätze (90a, b) versehen ist und die Öffnungen (84a, b) an die Kanäle (78a, 78b) angrenzen oder zu diesen ausgerichtet sind und das Einsetzen der länglichen Fortsätze (90a, b) und der Vorsprünge (92a, b) innerhalb der zylindrischen Wand in die zugehörigen länglichen Kanäle (78a, b) ermöglichen, wobei die Kanäle (78a, b) darin ausgebildete Seitenöffnungen (94a, b) zum lösbar verriegelnden Aufnehmen der Vorsprünge (92a, b) aufweisen, wenn das erste Ende (72a) einer Nadel (70) von dem Kragen (88) umgeben ist.

2. Halter (69) nach Anspruch 1, **dadurch gekennzeichnet,** daß die länglichen Fortsätze (90a, b) ein Paar relativ flacher, verlängerter Fortsätze zum Zusammenpassen mit den länglichen Kanälen (78a, 78b) und zum Gleiten darin aufweisen und mit den Kanälen (78a, 78b) in einem im wesentlichen gleitenden Eingriff stehen, um ein Vor- und Zurückgleiten der Fortsätze in den Kanälen zu gestatten, ohne das Aufnehmen eines standardmäßigen, vakuumdichten Fluidhalters in den Innenraum des Körpers (74) zu behindern.

3. Halter (69) nach Anspruch 1, **dadurch gekennzeichnet,** daß die seitlichen Öffnungen (94a, b) etwa in der Mitte zwischen dem geschlossenen und dem offenen Ende des Körpers (74) angeordnet und im wesentlichen rechteckig zum Aufnehmen der damit in Eingriff stehenden Vorsprünge (92a, b) von derselben etwa rechteckigen Gestalt ausgebildet sind.

## Revendications

1. Un support (69) pour aiguilles (70), ledit support (69) présentant une protection pour aiguille amovible (84), comprenant:
a. un protège-aiguille (84) en forme de U, comportant une plaque frontale annulaire (86) s'étendant radialement et présentant une ouverture axiale dans celle-ci, avec un collier (88) qui s'étend sur sa périphérie pour permettre à une première extrémité (72a) d'une aiguille (70) d'en dépasser, et des extensions longitudinales (90a, b) sur ledit collier (88) s'étendant vers l'arrière de celui-ci, et ayant des pattes (92a, b) aux extrémités de celui-ci s'étendant latéralement vers l'extérieur de celui-ci ;
b. un corps allongé (74) présentant une paroi cylindrique longitudinale et une extrémité fermée (80) dotée, sur celle-ci, d'un moyeu (82) pour recevoir de façon amovible ladite aiguille (70), et des ouvertures (84a, b) espacées radialement dudit moyeu, et une extrémité ouverte à distance de ladite extrémité fermée (80) ;
caractérisé en ce que le support (69) est un support de protection (69) adapté pour recevoir une aiguille à deux bouts (70) et la paroi cylindrique longitudinale étant adaptée pour recevoir et supporter un récepteur de fluide sous vide pouvant y être inséré de façon à permettre à une seconde extrémité de l'aiguille de pénétrer dans le récepteur de fluide pour transférer le fluide à travers l'aiguille vers ou depuis le récepteur de fluide, la paroi cylindrique étant dotée de canaux longitudinaux espacés (78a, 78b) pour recevoir de façon coulissante lesdites extensions longitudinales (90a, b) et lesdites ouvertures (84a, b) étant adjacentes ou alignées avec les canaux (78a, 78b) et permettant aux extensions longitudinales (90a, b) et aux pattes (92a, b) d'être insérées dans la paroi cylindrique à l'intérieur des canaux longitudinaux (78a, b) associés, lesdits canaux (78a, b) étant dotés à l'intérieur d'ouvertures latérales (94a, b) pour recevoir lesdites pattes (92a, b) pour les bloquer de façon déverrouillable lorsque ladite première extrémité (72a) d'une aiguille (70) est entourée par ledit collier (88).

2. Un support (69) selon la revendication 1, dans lequel les extensions longitudinales (90a, b) présentent une paire d'extensions allongées relativement plates pour raccorder et faire glisser à l'intérieur des canaux longitudinaux (78a, b) et présentant une configuration d'accouplement substantiellement coulissante avec lesdits canaux (78a, b) pour permettre aux dites extensions de coulisser vers l'arrière et vers l'avant à l'intérieur des canaux sans empêcher l'intérieur du corps (74) de recevoir un récepteur de fluide sous vide standard.

3. Un support (69) selon la revendication 1, dans lequel les ouvertures latérales (94a, b) sont disposées approximativement à midistance entre les extrémités fermée et ouverte du corps (74) et sont de forme substantiellement rectangulaire pour recevoir des pattes (92a, b) substantiellement de raccord de forme rectangulaire substantiellement similaire.
